# EUROPEAN PATENT APPLICATION

(11) **EP 2 143 418 A1**
(43) Date of publication of application: **13.01.2010**
(21) Application number: 09162777.8
(22) Date of filing: 16.06.2009
(51) Int. Cl.: A61K 8/64, A61Q 15/00

(54) **Antiperspirant compositions**

(30) Priority: 08.07.2008 EP 08159876; 08.07.2008 EP 08159877
(71) Applicant: Unilever PLC, 100 Victoria Embankment London EC4Y 0DY (GB)
(72) Inventor: Courtois, Jean-Philippe Andre Roger, Merseyside CH63 3JW (GB); Harding, Clive Roderick, Merseyside CH63 3JW (GB); Harker, Mark, Merseyside CH63 3JW (GB); Kelso, Hailey, Merseyside CH63 3JW (GB); Weddell, Iain Andrew, Merseyside CH63 3JW (GB)
(74) Representative: Whaley, Christopher

(57) **Abstract**

A non-therapeutic method of reducing perspiration comprising the topical application of a lamellar phase stabilised oil-in-water emulsion and a peptide having antiperspirant properties to the surface of the body and compositions suitable therefor.

## Description

This invention relates to the field of cosmetic products, in particular antiperspirant products. This invention also relates to methods of reducing perspiration upon the surface of the human body.

Cosmetic compositions used for reducing perspiration often comprise an astringent metal salt, such as aluminium or zirconium salt. Such salts are perceived as harsh or irritating by some consumers and there is a need for alternative means of achieving sweat reduction.

There are also disclosures in the prior art of compositions that reduce perspiration without the use of such astringent metal salts. In general, however, these compositions have not been particularly effective. Some such compositions comprise antiperspirant agents that act at a sub-cellular level, decreasing sweat at its source: the secretory coil of the sweat glands. Such agents generally have an effect upon the eccrine glands, but they may also affect the apocrine glands.

Publications have recently appeared suggesting that certain peptides might be effective against hyperhidrosis, or excessive sweating, by acting at a sub-cellular level within the secretory coil of the sweat glands.

WO 06/094193 (Revance Therapeutics Inc.) discloses multiple uses of peptides comprising the sequence Glu.Glu.Met.Gln.Arg.Arg., one of said uses being the treatment of hyperhidrosis.

A further peptide derivative, commercially available from Pentapharm AG and having the structure: β-Ala-Pro-diaminobutyroylbenzylamide, is disclosed as suitable for treating hyperhydrosis in Research Disclosure, Vol. 519, 07 2007, p. 685.

There is a continuing need for effective perspiration reduction, particularly by agents that are not perceived as harsh or irritating. The problem to be solved is the provision of effective or improved sweat control *via* the use of a cosmetically acceptable composition or compositions.

In a first aspect of the present invention, there is provided a cosmetic composition comprising a lamellar phase stabilised oil-in-water emulsion and a peptide having antiperspirant properties.

In a second aspect of the present invention, there is provided a non-therapeutic method of reducing perspiration comprising the topical application of a lamellar phase stabilised oil-in-water emulsion and a peptide having antiperspirant properties to the surface of the body.

In a third aspect of the present invention, there is provided the use of a lamellar phase stabilised oil-in-water emulsion and a peptide having antiperspirant properties for the manufacture of a composition for the treatment of hyperhidrosis.

In the second aspect of the invention, it is not essential that the lamellar phase stabilised oil-in-water emulsion and the peptide having antiperspirant properties are part of the same composition. Independent application of the two materials may be employed. Such application may be concurrent or consecutive, provided that the treated surface experiences the presence of both components. When applied from independent compositions, it is preferred that the product also comprises a means for, and/or instruction for, both of the compositions to be applied to the surface requiring treatment.

The method of controlling perspiration offered by the invention is particularly useful because the benefit can extend for a considerable period of time, often greater than 24 hours and sometimes even up to 4 days after application or longer. Significant deodorancy benefits may also accrue from the use of the present invention.

The method of controlling perspiration and compositions according to present invention are particularly useful when direct application to the surface of the human body is involved. This is especially true when application to the axillae and/or feet is involved. Application to the axillae is most preferred because of the high concentration of eccrine sweat glands in these regions of the human body.

The method of controlling perspiration typically involves multiple applications of the composition or compositions. It is thought that multiple applications improve perspiration reduction by some form of build up effect. The composition or compositions may be applied as part of one's daily regime, after washing, for example.

In the methods and compositions according to the present invention, it believed that the lamellar phase stabilised oil-in-water emulsion surprisingly enhances the antipersprancy performance of the peptide.

In this description, the term "comprising" should be understood to be non-exhaustive, i.e., meaning that other components may also be present.

Lamellar phase stabilised oil-in-water emulsions as used in the present invention may be called oleosome dispersions. Typically, they comprise dispersed droplets of oil surrounded by multiple layers of liquid crystalline lamellar phase comprised of one or more surfactants, non-ionic surfactants being preferred.

The lamellar phase stabilised oil-in-water emulsion typically forms part of a cosmetic composition. Further features of such compositions are described in the following paragraphs.

The oil of the oleosome dispersions should preferably remain in a liquid state at temperatures as low as 20°C or even 15°C. For this reasons, typical oils have melting point from their solid state of less than 20°C, preferably less than 15°C, and more preferably less than 10°C.

Preferred oils should be relatively fluid, having a kinematic viscosity of less than 100 cSt (mm²/s) and preferably less than 50 cSt (mm²/s) at 20°C.

Suitable oils include hydrocarbon oils and ester oils, particularly triglyceride oils, such as sunflower seed oil. Other particular oils that might be used in the oleosomes are C₁₂₋₁₅ alkyl benzoate esters, hydrogenated polybutene, PPG-14 butyl ether, triethyl citrate, isopropyl palmitate, and isopropylmyristate.

The lamellar phase of the oleosome dispersion is typically comprised of two non-ionic surfactants, one having a relatively low HLB (less than 8 and preferably less than 5) and one having a relatively high HLB (more than 12 and preferably more than 15). The ratio high HLB surfactant to low HLB surfactant is chosen so as to give a stable oleosome dispersion, typically ratios being from 1:1 to 1:6 by weight, respectively. A blend of steareth-2 and steareth-20 has been found to be suitable, particularly when used in combination with sunflower oil. Steareth-2 and steareth-20 are best used at a ratio of from 1:4 to 1:5 by weight.

The oleosome dispersions have an oil content that typically makes up from 1 to 30%, preferably from 1 to 20%, and more preferably from 1 to 10% by weight of the total composition. The surfactant content typically makes up from 1 to 30%, preferably from 1 to 20%, and more preferably from 1 to 10% by weight of the composition. The ratio of oil to surfactant is typically from 1:3 to 3:1 and preferably from 1:2 to 2:1.

The peptide having antiperspirant properties used in accordance with the present invention may be any peptide capable of reducing perspiration when applied to the surface of the body.

It is highly preferred that the peptide is water soluble. In this description, the term "water soluble" should be understood to refer to materials having a solubility in water at 25°C of 1 g/L or greater. Preferred peptides have a water solubility of 5g/L or greater and particularly preferred peptides have a water solubility of 10g/L or greater.

It is preferred that the peptide is of 12 amino acid residues or less, more preferably 10 amino acid residues or less, and most preferably 8 amino acid residues or less. It has been found that longer peptides have at least the potential for causing undesirable responses on application to the human body.

The stereochemistry of the amino acids of the peptide is typically L-.

A particular group of peptides capable of reducing perspiration comprise the amino acid sequence Glu.Glu.Met.Gln.Arg.Arg. Other amino acids may also be present in the peptide, provided that they do not interrupt the above sequence.

A particularly suitable peptide is the hexapeptide Glu.Glu.Met.Gln.Arg.Arg. This hexapeptide is commercially available as an acetate derivative under the trade name Argireline^{®}. The structures of this material may be represented as: Ac-Glu-Glu-Met-Gln-Arg-Arg-NH₂ or Ac-EEMQRR-NH₂.

It is preferred that the peptide capable of reducing perspiration does not comprise the amino acid sequence Glu.Glu.Met.Gln.Arg.Arg.Ala. In other words, it is preferred that compositions according to the invention do not comprise a peptide comprising the amino acid sequence Glu.Glu.Met.Gln.Arg.Arg.Ala.

A particular group of peptides capable of reducing perspiration comprise the amino acid sequence Tyr.Ala.Gly.Phe.Leu. Other amino acids may also be present in the peptide, provided that they do not interrupt the above sequence. Peptides of this type are disclosed in WO 06/106164 (Lipotec S.A.). A peptide of this type having the structure Tyr.Ala.Gly.Phe.Leu. is commercially available under the trade name Leuphasyl^{®} from Lipotec SA.

A particular peptide capable of reducing perspiration has the structure β-Ala-Pro-diaminobutyroylbenzylamide. The name of this peptide may be abbreviated to β-Ala-Pro-Dab. This peptide is commercially available under the trade name Syn-Ake^{®} from Pentapharm AG. This peptide and others potentially capable of reducing perspiration are disclosed in WO 06/047900 (Pentapharm AG).

In this description, the term "peptide" should be understood to mean "peptide or derivative thereof". "Derivatives thereof" are typically esters, amides, or salts. Acetate esters are a particular example. Certain peptide derivatives hydrolyse on application to yield the parent peptide.

The peptide is preferably incorporated into a composition in an amount of from 0.0005 to 1%, particularly from 0.001 to 0.5%, and especially from 0.005 to 0.2% by weight of the composition. These levels of incorporation are extremely low when compared to conventional antiperspirant agents and allow space for significant levels of other components within compositions for use in accordance with the present invention (*vide infra*).

An anti-microbial deodorant agent is a preferred additional component of compositions suitable for use in accordance with the present invention. The anti-microbial deodorant agent may be a bacteriocidal agent or a bacteriostatic agent. Synergistic deodorancy benefits may be achieved using compositions comprising an anti-microbial deodorant agent.

Preferred anti-microbial deodorant agents are organic in nature and such anti-microbial deodorant agents are particularly preferred in compositions that do not comprise an astringent salt of aluminium and/or zirconium.

When employed, the anti-microbial deodorant agent is typically incorporated into the composition at from 0.01 % to 3% and particularly at from 0.03% to 0.5%.

Preferred anti-microbial deodorant agents have a minimum inhibitory concentration (MIC) of 1 mg.ml⁻¹ or less, particularly 200 µg.ml⁻¹ or less, and especially 100 µg.ml⁻¹ or less. The MIC of an anti-microbial agent is the minimum concentration of the agent required to significantly inhibit microbial growth. Inhibition is considered "significant" if an 80% or greater reduction in the growth of an inoculum of *Staphylococcus epidermidis* is observed, relative to a control medium without an anti-microbial agent, over a period of 16 to 24 hours at 37°C. Details of suitable methods for determining MICs can be found in "Antimicrobial Agents and Susceptibility Testing", C.Thornsberry, (in "Manual of Clinical Microbiology", 5th Edition, Ed. A. Balows et al, American Society for Microbiology, Washington D.C., 1991). A particularly suitable method is the Macrobroth Dilution Method as described in Chapter 110 of above publication (pp. 1101-1111) by D. F. Sahm and J. A. Washington II. MICs of anti-microbials suitable for inclusion in the compositions of the invention are triclosan: 0.01-10 µg.ml⁻¹ (J.Regos et al., Dermatologica (1979), 158: 72-79) and farnesol: ca. 25 µg.ml⁻¹ (K. Sawano, T. Sato, and R. Hattori, Proceedings of the 17th IFSCC International Conference, Yokahama (1992) p.210-232). By contrast ethanol and similar alkanols have MICs of greater than 1 mg.ml⁻¹.

Suitable organic anti-microbials are bactericides, for example quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred anti-microbials for use in the compositions of the invention are polyhexamethylene biguanide salts (also known as polyaminopropyl biguanide salts), an example being Cosmocil CQ™ available from Zeneca PLC, preferably used at up to 1% and more preferably at 0.03% to 0.3% by weight; 2',4,4'-trichloro,2-hydroxy-diphenyl ether (triclosan), preferably used at up to 1 % by weight of the composition and more preferably at 0.05-0.3%; and 3,7,11-trimethyldodeca-2,6,10-trienol (farnesol), preferably used at up to 1% by weight of the composition and more preferably at up to 0.5%.

Other suitable organic antimicrobial agents are transition metal chelators, as described in WO01/52805, for example. Transitional metal chelators having a binding coefficient for iron(III) of greater than 10²⁶, for example diethylenetriaminepentaacetic acid and salts thereof are preferred.

The lamellar phase stabilised oil-in-water emulsion may be applied to the surface of the human body by any means. Liquid compositions comprising the lamellar phase stabilised oil-in-water emulsion can be by absorption onto a carrier matrix like paper, fabric, or sponge and applied by contacting said carrier matrix with the surface. Application can also comprise a combination of any two or more of the above techniques.

Other cosmetically acceptable components may also be present in compositions used in accordance with the present invention. Such will vary according to the desired mode of application of the composition. For example, a volatile propellant is a typical component in compositions for spray application and a liquid carrier fluid (usually water) is a typical component in compositions for roll-on application. Thickeners, in particular cellulosic thickeners such as hydroxypropylcellulose and hydroxyethycellulose may also be used in roll-on compositions.

Certain sensory modifiers are further desirable components. Such materials are preferably used at a level of up to 20% by weight of the composition. Emollients, humectants, volatile oils, non-volatile oils, and particulate solids which impart lubricity are all suitable classes of sensory modifiers. Examples of such materials include cyclomethicone, dimethicone, dimethiconol, isopropyl myristate, isopropyl palmitate, talc, finely-divided silica (eg. Aerosil 200), particulate polyethylene (eg. Acumist B18), polysaccharides, corn starch, C12-C15 alcohol benzoate, PPG-3 myristyl ether, octyl dodecanol, C7-C14 isoparaffins, di-isopropyl adipate, isosorbide laurate, PPG-14 butyl ether, glycerol, hydrogenated polyisobutene, polydecene, titanium dioxide, phenyl trimethicone, dioctyl adipate, and hexamethyl disiloxane.

Fragrance is also a desirable additional component. Suitable materials include conventional perfumes, such as perfume oils and also include so-called deoperfumes, as described in EP 545,556 and other publications. Levels of incorporation are preferably up to 4% by weight, particularly from 0.1% to 2% by weight, and especially from 0.7% to 1.7% by weight.

It should be noted that certain components of compositions perform more than one function. Such components are particularly preferred additional ingredients, their use often saving both money and formulation space. Examples of such components include ethanol, isopropyl myristate, and the many components that can act as sensory modifiers.

Further additional components that may also be included are colourants and preservatives at a conventional concentration, for example C₁-C₃ alkyl parabens.

### Examples

The compositions indicated in Table 1 were prepared using methods known in the art. The steareth-2, steareth-20, and sunflower oil formed a lamellar phase stabilised oil-in-water emulsion.

Throughout these Examples, percentages are by weight, unless otherwise indicated.

**Table 1**

| **Chemical Name** | **Amount (%)** | | | **Trade Name** | **Supplier** |
|---|---|---|---|---|---|
| **Composition:** | **1** | **2** | **3** | | |
| Water and minors | 82.8 | 88.8 | 72.8 | | Local |
| Helianthus Annuus¹ | 4.0 | 4.0 | 4.0 | Agri Pure 80 | Cargill |
| Steareth-2 | 2.6 | 2.6 | 2.6 | Volpo S2 | Croda |
| Steareth-20 | 0.6 | 0.6 | 0.6 | Brij 78 | Uniqema |
| β-Ala-Pro-Dab | -- | 4³ | -- | Syn-Ake® | Pentapharm |
| Tyr.Ala.Gly.Phe.Leu. | 10² | -- | -- | Leuphasyl® | Lipotec SA |
| Ac-EEMQRR-NH₂ | -- | -- | 20⁴ | Argireline® | Lipotec SA |

| | | | | | |
|---|---|---|---|---|---|
| 1. Sunflower seed oil. 2. This amount refers to the 0.05% aqueous solution of Leuphasyl^{®} supplied by Lipotec. Hence, the actual amount of peptide present in the composition was 0.005%. 3. This amount refers to the 0.25% aqueous-glycerol (70/30 w/w) solution of Syn-Ake^{®} supplied by Pentapharm. Hence, the actual amount of peptide present in the composition was 0.01 %. 4. This amount refers to the 0.05% aqueous solution of Argireline^{®} supplied by Lipotec. Hence, the actual amount of peptide present in the composition was 0.01 %. | | | | | |

The following protocol was used to evaluate the compositions of Table 1. Trained operators applied one composition to a first axilla of panellists once each day for four consecutive days in week one and then for three consecutive days in week two (ca. 0.3g per axilla). A control composition was applied to the panellists' second axilla. The panellists self-applied at home at the weekend in between the two test weeks. A conventional roll-ball applicator was used for the applications. At intervals during the test, panellists were required to sit in a hot room (temperature: 40°C; relative humidity: 40%) for up to one hour and twenty minutes. During the sittings, sweat was collected on absorbent cotton pads held in each axilla and the amount produced was recorded.

Hot room sittings were done after the periods indicated in Table 2. The final sitting was done 96 hours after the final applications, the panellists being allowed to wash as normal during the intervening period. Table 2 indicates the "Sweat Weight Reduction (SWR)" achieved by each composition relative to the control composition (which was a simple ethanolic deodorant, lacking any antiperspirant active).

**Table 2**

| **Time*** | **SWR (%)** | | |
|---|---|---|---|
| **Composition:** | **1** | **2** | **3** |
| 4 days | -- | 16.5 | -- |
| 7 days | -- | 26.5 | 22.6 |
| 10 days | 25.4 | 27.5 | 27.7 |
| + 4 days "wash out" | 18 | 18 | 17.9 |

| | | | |
|---|---|---|---|
| * Time after initial application. Hot room sittings done before application on days 4 and 7. | | | |

Significant SWRs were achieved with all three compositions. The results after the 4 day "wash out" period were particularly surprising.

In a further study, the Argireline® (at 0.01 %, as in Composition 2) was formulated into a simple aqueous ethanol roll-on composition thickened by hydroxypropylcellulose and was evaluated using the protocol described above. The compositions are indicated in Table 3.

**Table 3**

| **Chemical Name** | **Amount (%)** | | **Trade Name** | **Supplier** |
|---|---|---|---|---|
| **Composition:** | **A** | **B** | | |
| Water | 79.4 | 59.4 | | |
| Ac-EEMQRR-NH₂ | -- | 20.0¹ | Argireline® | Lipotec SA |
| Alcohol | 20 | 20 | Ethanol | BDH |
| Hydroxypropylcellulose | 0.6 | 0.6 | Klucel M | Hercules |

| | | | | |
|---|---|---|---|---|
| 1. This amount refers to the 0.05% aqueous solution of Argireline^{®} supplied by Lipotec. Hence, the actual amount of peptide present in the composition was 0.01 %. | | | | |

Composition B (with Argireline®) only reached a SWR of 6.4% after 10 days, relative to Composition A (without Argireline®). Clearly, this difference is much less than that achieved for the Examples according to the invention and, indeed, it was not significant at the 95% level.

## Claims

1. A non-therapeutic method of reducing perspiration comprising the topical application of a lamellar phase stabilised oil-in-water emulsion and a peptide having antiperspirant properties to the surface of the body.

2. A method according to claim 1, wherein the peptide comprises the amino acid sequence Glu.Glu.Met.Gln.Arg.Arg.

3. A method according to claim 1 or claim 2, wherein the peptide does not comprise the amino acid sequence Glu.Glu.Met.Gln.Arg.Arg.Ala.

4. A method according to claim 1, wherein the peptide comprises the amino acid sequence Tyr.Ala.Gly.Phe.Leu.

5. A method according to claim 1, wherein the peptide is β-Ala-Pro-diaminobutyroylbenzylamide.

6. A method according to any of preceding claims, wherein the lamellar phase stabilised oil-in-water emulsion comprises oil droplets surrounded by multiple layers of liquid crystalline phase comprised of one or more surfactants.

7. A method according to claim 6, wherein the multiple layers of liquid crystalline phase comprise non-ionic surfactant.

8. A method according to claim 7, wherein the multiple layers of liquid crystalline phase comprise two non-ionic surfactants, one having a HLB of less than 8 and another having a HLB of greater than 12.

9. A composition comprising a lamellar phase stabilised oil-in-water emulsion and a peptide having antiperspirant properties.

10. A composition according to claim 1, comprising an anti-microbial deodorant agent.

11. The use of a lamellar phase stabilised oil-in-water emulsion and a peptide having antiperspirant properties for the manufacture of a composition for the treatment of hyperhidrosis.
